# EUROPÄISCHE PATENTANMELDUNG

(11) **EP 2 329 799 A1**
(43) Veröffentlichungstag der Anmeldung: **08.06.2011**
(21) Anmeldenummer: 10190656.8
(22) Anmeldetag: 10.11.2010
(51) Int. Cl.: A61F 2/84

(54) **Kathetersystem mit Werkzeug zum Aufschneiden der Katheterhülle**

(30) Priorität: 07.12.2009 US 267116 P
(71) Anmelder: Biotronik VI Patent AG, 6341 Baar (CH)
(72) Erfinder: Fargahi, Amir, 8180, Bülach (CH)
(74) Vertreter: Lindner-Vogt, Karin L.

(57) **Zusammenfassung**

Kathetersystem bestehend aus einem Katheter mit einem distalen und einem proximalen Ende; einem Funktionsabschnitt, der nahe dem distalen Ende des Katheters liegt; einem Innenschaft mit Führungsdrahtlumen; einem Außenschlauch, wobei der Außenschlauch den Funktionsabschnitt vollständig oder teilweise umschließt; einem Führungsdrahtausgang mit distalem und proximalem Teilstück, wobei der Führungsdrahtausgang durch den Innenschaft und den Außenschlauch führt; einem Führungsdraht, wobei sich der Führungsdraht am distalen Ende des Katheters innerhalb des Führungsdrahtlumens befindet, und durch den Führungsdrahtausgang aus dem Katheter herausgeführt wird, wobei der Führungsdrahtausgang nahe dem distalen Ende des Katheters und proximal vom Funktionsabschnitt angebracht ist; wobei an dem distalen Teilstück des Führungsdrahtausgangs eine Schneidevorrichtung zum Durchtrennen des Außenschlauchs beim Zurückziehen desselben angebracht ist.

## Beschreibung

Die vorliegende Erfindung bezieht sich auf ein Kathetersystem mit einem Innenschaft, der ein Führungsdrahtlumen umgibt, und einem Außenschlauch, wobei der Außenschlauch einen Funktionsabschnitt aufweist.

### Hintergrund der Erfindung

Katheter, insbesondere drahtgeführte Katheter, haben eine breite Verwendung bei vorwiegend medizinischen Anwendungen und Eingriffen gefunden. Insbesondere in der Angioplastie und bei der Implantation von Stents werden drahtgeführte Katheter eingesetzt. Bei derartigen Anwendungen wird der Katheter mit Hilfe eines vorher in den Körper des Patienten eingeführten Führungsdrahts positioniert. Die distale Spitze eines Katheter-Führungsdrahts ist in der Regel sehr flexibel ausgebildet, so dass diese gebogen und gedreht werden kann, um zu der gewünschten Stelle im Körper des Patienten, z. B. innerhalb eines Blutgefäßes, vorgeschoben werden zu können. Es sind verschiedenste drahtgeführte Kathetertypen bekannt, so beispielsweise interventionelle Katheter, Ballonkatheter und Katheter zur Applikation von selbstexpandierenden Stents.

Bei so genannten Monorail-Kathetern, die auch als Rapid-Exchange-Katheter bezeichnet werden, läuft der Führungsdraht im Gegensatz zu Over-the-wire-Kathetern nicht auf der gesamten Länge im Führungsdrahtlumen des Katheters entlang, sondern wird nahe dem distalen Ende des Katheters aus dem Führungsdrahtlumen herausgeführt. Zu diesem Zweck hat der Katheter an der entsprechenden Stelle einen Führungsdrahtausgang. Entsprechende Anordnungen sind zum Beispiel beschrieben in US 5,290,241 und US 5,324,269 und US 6,251,084.

Eine im Stand der Technik bekannte Anordnung besteht aus einem Kathetersystem mit Katheter und Führungsdraht. Der Katheter weist nahe seinem distalen Ende einen Führungsdrahtausgang für den Austritt des Führungsdrahtes aus dem Katheter-Führungsdrahtlumen auf. Proximal von dem Führungsdrahtausgang weist der Außenschaft des Katheters eine Art Rille oder Vertiefung auf. Diese Rille oder Vertiefung dient dazu, den Führungsdraht beim Zurückziehen des Außenschlauches aufzunehmen. Da beim Zurückziehen des Außenschlauches dieser sich auf den Katheter presst und gleichzeitig den Führungsdraht einklemmt, muss für den Führungsdraht Platz geschaffen werden.

Um ein Kathetersystem herzustellen, das möglichst flexibel, mit kleinem Durchmesser und daher schonend für den Patienten ist, werden Katheter und Außenschlauch mit möglichst kleinem Durchmesser angefertigt. Das erzeugt zwangsläufig bei Bewegungen beider gegeneinander eine erhöhte Reibung. Eine weitere Erhöhung des Reibungseffekts wird hervorgerufen, wenn der Führungsdraht - wie in den herkömmlichen Lösungen - zwischen Außenschlauch und Katheter eingeklemmt wird. Diese Reibung wird nochmals verstärkt, da der Führungsdraht gemeinhin aus einem anderen Material besteht als Katheter und Außenschlauch.

Die vorbekannten Lösungen haben darüber hinaus den Nachteil, dass im Falle einer Torsion oder Biegung des Katheters im Körper des Patienten der Prozess der Stentfreigabe gestört oder sogar verhindert wird. Im Falle einer Torsion des Katheters ist ebenfalls die Rille für die Aufnahme des Führungsdrahtes bei Zurückziehen des Außenschlauchs tordiert. Das bewirkt, dass sich der Führungsdraht nicht in die dafür vorgesehene Rille legt, sondern deren Ränder überschreitet und in dem nicht vertieften Bereich des Katheters zum Liegen kommt. Dadurch wird der Führungsdraht zwischen Katheter und Außenschlauch eingeklemmt und erschwert, wenn nicht gar verhindert das Zurückziehen des Außenschlauchs; der Stent kann nicht freigesetzt werden.

Ein weiterer Nachteil der vorbekannten Rillen-Lösung ist die Versteifung des Katheters. In den ursprünglich runden Querschnitt eines Katheters wird durch eine Rille eine Vertiefung über die gesamte Katheterlänge eingelassen. Eine derartige Formveränderung bewirkt eine höhere Steifigkeit des Katheters. Durch die höhere Steifigkeit und die damit einhergehende geringere Flexibilität ist das Handling für den behandelnden Arzt erschwert und kann zu Komplikationen führen, unter denen der Patient leidet.

Die Aufgabe der vorliegenden Erfindung besteht somit darin, ein Kathetersystem zur Verfügung zu stellen, das die oben genannten Nachteile eliminiert oder zumindest verringert. Insbesondere soll die Reibung zwischen dem Führungsdraht und dem Außenschlauch bei Zurückziehen desselben, also bei der so genannten Pullback-Bewegung, verringert werden.

Die Aufgabe wird erfindungsgemäß durch die Merkmale des unabhängigen Anspruchs gelöst. Günstige Ausgestaltungen und Vorteile der Erfindung ergeben sich aus den weiteren Ansprüchen und der Beschreibung.

Die Erfindung geht aus von einem Kathetersystem, das aus einem Katheter, einem Funktionsabschnitt, einem Innenschaft mit Führungsdrahtlumen, einem Außenschlauch und einem Führungsdrahtausgang und einem Führungsdraht besteht. Der Führungsdrahtausgang ist mit einer Schneidevorrichtung zum Durchtrennen des Außenschlauchs beim Zurückziehen desselben versehen.

Durch das Zurückziehen des Außenschlauchs wird der Funktionsabschnitt freigegeben. Dadurch, dass dabei der Außenschlauch aufgetrennt wird, wird die Reibung zwischen dem Führungsdraht und dem Außenschlauch bei Zurückziehen desselben, also bei der so genannten Pullback-Bewegung, verringert. Durch das Auftrennen des Außenschlauchs wird Platz geschaffen für den Führungsdraht, der beim Zurückziehen des Außenschlauchs nicht zwischen Katheter und Außenschlauch zum Liegen kommt, sondern weiterhin außerhalb des Katheters liegt.

Weiterhin werden durch das Auftrennen des Außenschlauchs am Führungsdrahtausgang mögliche Komplikationen auf Grund einer Torsion des Katheters vermieden. Dadurch, dass der Führungsdraht während und nach dem Zurückziehen des Außenschlauchs sich außerhalb des Katheters befindet, bleibt er unbeeinträchtigt von einer Torsion des Katheters und erschwert das Zurückziehen das Außenschlauchs selbst bei vorhandener Katheter-Torsion nicht.

Bei dem erfindungsgemäßen Kathetersystem kommt es weiterhin zu keiner baubedingten Versteifung des Katheters. Dadurch, dass der Außenschlauch aufgetrennt wird und damit Platz für den Führungsdraht beim Zurückziehen des Außenschlauchs außerhalb des Katheters schafft, ist keine Rille oder anderweitige Vertiefung im Katheter notwendig, die eine baubedingte Versteifung des Katheters zur Folge hätte.

Der Katheter weist ein distales und ein proximales Ende auf. Axial durch den Katheter zieht sich über seine gesamte Länge das Führungsdrahtlumen, was von dem Innenschaft eingefasst wird. Ein Führungsdrahtausgang befindet sich nahe dem distalen Ende des Katheters und proximal vom Funktionsabschnitt. Der Führungsdraht, der frei beweglich ist bis durch die Katheterspitze, befindet sich am distalen Ende des Katheters innerhalb des Führungsdrahtlumens und wird durch den Führungsdrahtausgang aus dem Katheter herausgeführt wird. Der Führungsdrahtausgang besteht aus einer durch den Innenschaft und den Außenschlauch durchgehenden Perforation. Er weist ein distales und ein proximales Teilstück auf. Erfindungsgemäß befindet sich am distalen Teilstück des Führungsdrahtausgangs eine Schneidevorrichtung zum Durchtrennen des Außenschlauchs.

Die Materialien für den Außenschlauch und den Innenschaft sind aus dem Stand der Technik entnehmbar. So kann der Außenschlauch z. B. bestehen aus einem Kunststoff (bspw. PA, PEBA, PUR, Teflon) oder aus einem mehrschichtigen Kunststoff. Bei einem mehrschichtigen Aufbau besteht die Außenschicht aus festen Materialien wie zum Beispiel PA, PEBA, PUR und die aus Innenschicht aus reibungsarmen Materialien wie zum Beispiel HDPE, Teflon. Darüber hinaus kann der Außenschlauch eine mittlere Schicht aufweisen, die entweder als Haftvermittler oder als Verstärkung dient. Als Verstärkung bietet sich zum Beispiel ein Metalldraht, ein Metallcoil oder ein Metallbraid an.

Am Ort der Behandlung wird der Außenschlauch, der während des Implantationsvorgangs den Funktionsabschnitt ganz oder teilweise umschlossen hat, zurückgezogen und setzt den Funktionsabschnitt frei. Beim Zurückziehen des Außenschlauchs trifft dieser auf die Schneidevorrichtung am distalen Teilstück des Führungsdrahtausgangs und wird dort aufgetrennt. Dadurch entsteht eine Lücke im Außenschlauch, die Platz für den Führungsdraht lässt.

Die Schneidevorrichtung, die bevorzugt als Klinge ausgestaltet ist, kann aus einem oder mehreren Metallen bestehen, wie z. B. aus rostfreiem Stahl, Co-Cr-Legierungen, Nitinol-Legierungen usw. oder aus einem oder mehreren keramischen Stoffen, wie z. B. Zirkonat.

Der Führungsdrahtausgang und die Schneidevorrichtung können getrennt voneinander am Katheter angebracht sein oder aber als zusammenhängendes Bauteil ausgestaltet sein. Materialien für beide Varianten wären beispielsweise Metalle, wie z. B. rostfreier Stahl, eine Co-Cr-Legierung oder eine Nitinol-Legierung. Beide können aber auch aus einem Spritzgussteil bestehend aus Materialien wie beispielsweise Polycarbonat, Polystyrene, ABS oder K-Resin gegossen sein. Ebenfalls kann das Material ein thermoplastischer Kunststoff wie z. B. PA, PEBA, PUR oder Silikon sein.

Der Funktionsabschnitt am distalen Ende des Katheters kann beispielsweise ausgelegt sein als Stentaufnahmeabschnitt oder als Ballonabschnitt.

In einer bevorzugten Ausführungsform ist der Funktionsabschnitt als Stentaufnahmeabschnitt ausgelegt. Stents werden derzeit weit verbreitet eingesetzt, da sie eine einfache und kostengünstige Behandlung von Gefäßerkrankungen ermöglichen. Sie weisen häufig ein rohrförmiges oder hohlzylinderförmiges Grundgitter auf, das an beiden Längsenden offen ist. Das Grundgitter einer derartigen Endoprothese wird mittels eines Katheters in den zu behandelnden Körperhohlraum eingesetzt und dient nach Entfernen des Katheters dazu, den Körperhohlraum zu stützen. Durch den Einsatz von Stents können verengte Bereiche in den Gefäßen dauerhaft oder zumindest über einen bestimmten Zeitraum erweitert werden, so dass ein Lumengewinn im Körperhohlraum resultiert.

Der für eine Implantation vorgesehene Stent ist angebracht am Funktionsabschnitt des Katheters. Der Stent kann ein beliebiger Stent, bekannt aus dem Stand der Technik, sein. Der Stent kann mit einem Medikament beschichtet oder aber unbeschichtet sein. Er kann in einer selbstexpandierenden oder ballondilatierbaren Variante vorliegen. Ebenfalls kann es sich um einen permanenten oder biodegradierbaren Stent handeln.

Intraluminale Endoprothesen werden heute häufig mit pharmazeutisch aktiven Substanzen versehen, die über einen bestimmten Zeitraum im Organismus freigesetzt werden. Diese pharmazeutisch aktiven Substanzen können beispielsweise dazu dienen, Restenosen oder Agglomerationen zu vermeiden. Durch die Freisetzung von pharmazeutisch aktiven Substanzen, mit denen derartige intraluminale Endoprothesen versehen sind, ist es möglich, lediglich eine lokale Behandlung, d. h. eine Elution eines Wirkstoffs im Wesentlichen nur in das die intraluminale Endoprothese umgebende Gewebe, vorzunehmen. Dieser Vorgang wird auch als "Local Drug Delivery" (LDD) bezeichnet. Der Behandlungsort, an dem der Wirkstoff seine pharmakologische Wirkung entfalten soll, grenzt somit unmittelbar an den Ort der Implantation der intraluminalen Endoprothese.

Unter einer "pharmazeutisch aktiven Substanz" (oder therapeutisch aktive oder wirksame Substanz) im Sinne der Erfindung wird ein pflanzlicher, tierischer oder synthetischer Wirkstoff (Medikament) oder ein Hormon verstanden, das in geeigneter Dosierung als Therapeutikum zur Beeinflussung von Zuständen oder Funktionen des Körpers, als Ersatz für natürlich vom menschlichen oder tierischen Körper erzeugte Wirkstoffe, wie Insulin, sowie zur Beseitigung oder zum Unschädlichmachen von Krankheitserregern, Tumoren, Krebszellen oder Körperfremdstoffen Einsatz findet. Die Freisetzung der Substanz in der Endoprothesenumgebung hat einen positiven Effekt auf den Heilungsverlauf oder wirkt pathologischen Veränderungen des Gewebes in Folge des chirurgischen Eingriffs entgegen bzw. dient dem Unschädlichmachen von maladen Zellen in der Onkologie.

Derartige pharmazeutisch aktive Substanzen weisen beispielsweise eine antünflammatorische und/oder antiproliferative und/oder spasmolytische Wirkung auf, wodurch beispielsweise Restenosen, Entzündungen oder (Gefäß-)Spasmen vermieden werden können. Derartige Substanzen können in besonders bevorzugten Ausführungsbeispielen aus einer oder mehreren Substanzen der Wirkstoffgruppe der Calciumkanalblocker, der Lipidregulatoren (wie beispielsweise Fibrate), der Immunsuppressiva, der Calcineurininhibitoren (wie beispielsweise Tacrolimus), der Antiflogistika (wie beispielsweise Cortison oder Dichlofenac), der Antiinflammatorica (wie beispielsweise Imidazole), der Antiallergika, der Oligonucleotide (wie beispielsweise dODN), der Östrogene (wie beispielsweise Genistein), der Endothelbildner (wie beispielsweise Fibrin), der Steroide, der Proteine, der Hormone, der Insuline, der Zytostatika, der Peptide, der Vasodilatatoren (wie beispielsweise Sartane) und der antiproliferativ wirkenden Stoffe, beispielsweise Paclitaxel oder Sirolimus oder dessen Derivaten, bestehen.

Der Stent kann mit Funktionselementen versehen sein, die zumindest in einem Teil ihres Volumens eine verglichen mit dem Material des Grundgitters verschiedene Materialzusammensetzung aufweisen. Diese Funktionselemente dienen beispielsweise der Bestimmung der Position des Stents im Körper oder dem Freisetzen von Medikamenten.

Die Ermittlung der Position des Stents geschieht häufig mittels bildgebender Verfahren, beispielsweise mittels einer Röntgenstrahleinrichtung. Da die für das Grundgitter von derartigen Stents verwendeten Materialien in der Regel Röntgenstrahlung nur in geringem Maße absorbieren bzw. streuen, d. h. röntgen- oder radioluzent sind, werden die Stents häufig mit so genannten Röntgenmarkern versehen, die ein Material enthalten, das eine höhere Absorption der Röntgenstrahlen aufweist (röntgenopakes oder radioopakes Material).

Besonders bevorzugt ist ein selbstexpandierender Stent. Dieser kann entweder aus einer Formgedächtnislegierung, wie z.B. Nitinol, hergestellt sein oder ein selbstexpandierendes Stent-Design (z. B. Wallstent und/oder Coil-Design) aufweisen. Hier hat der Außenschlauch die Aufgabe, den Stent in seiner komprimierten Form zu halten. Darüber hinaus kann der Außenschlauch ein eventuell vorhandenes Medikament auf dem Stent vor Ablösen (z. B. durch Abrieb oder Diffusion) während der Implantation schützen. Bei Implantation eines selbstexpandierenden Stents reicht allein das Zurückziehen des Außenschlauchs und der Stent geht in seine ursprünglich eingeprägte dilatierte Stellung über. Er legt sich an die Gefäßwand und drückt die Stenose auf, so dass die zu behandelnde Stelle wiedereröffnet oder erweitert wird und der Strom der Körperflüssigkeit in dem Gefäß nicht mehr oder nicht mehr in dem zuvor vorliegenden Maße behindert wird. Beim Zurückziehen des Außenschlauchs wird dieser mit Hilfe der Schneidevorrichtung durchtrennt.

In einer weiteren bevorzugten Ausführungsform ist der Stent ballondilatierbar. Hier kann der Außenschlauch ein eventuell vorhandenes Medikament, das auf dem Stent angebracht ist (Coating), schützen oder aber den Stent fester und unverschiebbar auf dem Katheter halten. Der Außenschlauch kann den Stent ganz oder teilweise umschließen. Sobald der Außenschlauch zurückgezogen wird, wird dieser mit Hilfe der Schneidevorrichtung aufgetrennt. Auf dem Katheter ist unter dem Stent ein Ballon angebracht. Wird der Ballon ― durch Einleiten einer Flüssigkeit unter Druck ― dilatiert, expandiert der darüber liegende Stent, bis er die gewünschte aufgeweitete Form erreicht hat. Bei Ablassen der Flüssigkeit faltet sich der Ballon wieder zusammen und kann entfernt werden. Der Stent bleibt am Behandlungsort zurück.

Derzeit werden auch intraluminale Endoprothesen eingesetzt, die aus einem Material bestehen, das der Biodegradation unterliegt. Hierbei werden unter Biodegradation hydrolytische, enzymatische oder andere stoffwechselbedingte Abbauprozesse im lebenden Organismus verstanden, die vor allem durch die mit der Endoprothese in Kontakt gelangenden Körperflüssigkeiten verursacht werden und zu einer allmählichen Auflösung zumindest großer Teile der Endoprothese führen. Synonym zu dem Begriff Biodegradation wird häufig der Begriff Biokorrosion verwendet. Der Begriff Bioresorption umfasst die anschließende Resorption der Abbauprodukte durch den lebenden Organismus. Derartige biodegradierbare Materialien können aus Polymeren oder Metallen realisiert werden. Im Zusammenhang mit Stents ist auch die Abkürzung AMS (Absorbierbarer Metall Stent) gebräuchlich. Derartige Stents enthalten ein biodegradierbares Metall, vorzugsweise Magnesium und/oder eine Magnesiumlegierung.

Der als Stentaufnahmeabschnitt ausgelegte Funktionsabschnitt kann einen oder mehrere Stents aufnehmen. Zur Implantation mehrerer Stents während eines Kathetereingriffs kann das System derart ausgelegt sein, dass der Außenschlauch jeweils nur soweit zurückgezogen wird, bis ein Stent, und zwar beginnend mit dem am weitesten distal liegenden Stent, freigesetzt ist. Anschließend wird der Katheter bis zur nächsten zu behandelnden Stelle bewegt; dort wird der nächste Stent durch weiteres Zurückziehen des Außenschlauchs freigelegt usw. Der Außenschlauch wird dabei jeweils zum für die Freisetzung eines Stents benötigten Teil aufgeschnitten.

In einer weiteren bevorzugten Ausführungsform ist der Funktionsabschnitt als Ballonabschnitt ausgelegt. Der am Ballonabschnitt angebrachte Ballon wird am Behandlungsort dilatiert und weitet somit das verengte Gefäß auf. Gleichzeitig hat er die Aufgabe, ein Medikament am Behandlungsort abzugeben (medikamentenbeschichteter Ballon, drug eluting balloon). In der Zeit, in der der Ballon in der dilatierten Form im Gefäß vorliegt, berührt er die Gefäßwände und gibt ein oder mehrere Medikamente an die Gefäßwand ab. Ein über den Ballon gezogener Außenschlauch dient dem Schutz des Medikaments vor Ablösen (z. B. durch Abrieb oder Diffusion) während des Weges durch das Gefäßsystem bis zum Behandlungsort. Am Behandlungsort wird der Außenschlauch zurückgezogen und dabei durchtrennt. Anschließend wird der Ballon dilatiert und gibt gleichzeitig das Medikament ab. Nach Komprimierung des Ballons kann dieser mitsamt dem Kathetersystem aus dem Gefäß zurückgezogen werden.

In einer weiteren bevorzugten Ausführungsform ist über der Schneidevorrichtung eine Schutzvorrichtung angebracht. Diese Schutzvorrichtung dient dazu, während des gesamten Eingriffs das Gewebe und insbesondere die Gefäßwände zu schützen. Da die Schneidevorrichtung naturgemäß scharf geschliffen ist, besteht die Gefahr, bei dem geringsten Kontakt zum Gewebe dieses zu verletzen. Das gilt es natürlich komplett zu verhindern, damit der Operateur jegliche notwendige Bewegung des Kathetersystems frei ausführen kann, um den Behandlungsort zu erreichen.

Vorzugsweise ist die Schutzvorrichtung ausgelegt als eine Halbschale, die nur die Schneidevorrichtung abdeckt oder als Rohr, das den Katheter an der Stelle, an der die Schneidevorrichtung angebracht ist, komplett umgibt.

Die Erfindung wird anhand von Ausführungsbeispielen in Verbindung mit den Figuren näher erläutert.
- Fig. 1: zeigt schematisch das Kathetersystem, bei dem der Funktionsabschnitt als Stentaufnahmeabschnitt, mit einem selbstexpandierenden Stent, ausgelegt ist.
- Fig. 2: zeigt schematisch das Kathetersystem, bei dem der Funktionsabschnitt als Stentaufnahmeabschnitt, mit einem ballondilatierbaren Stent, ausgelegt ist.
- Fig. 3: zeigt schematisch das Kathetersystem, bei dem der Funktionsabschnitt als Ballonabschnitt ausgelegt ist.
- Fig. 4: zeigt schematisch die Schneidevorrichtung.
- Fig. 5: zeigt schematisch die Schneidevorrichtung in Kombination mit dem Führungsdrahtsausgang.
- Fig. 6: zeigt schematisch die Schutzvorrichtung in Form einer Halbschale.
- Fig. 7: zeigt schematisch die Schutzvorrichtung in Form eines Rohrs.

Die Figuren sind schematische Darstellungen der Erfindung. Sie bilden nicht spezifische Parameter der Erfindung ab. Weiterhin geben die Figuren lediglich typische Ausgestaltungen der Erfindung wieder und sollen die Erfindung nicht auf die dargestellten Ausgestaltungen beschränken.

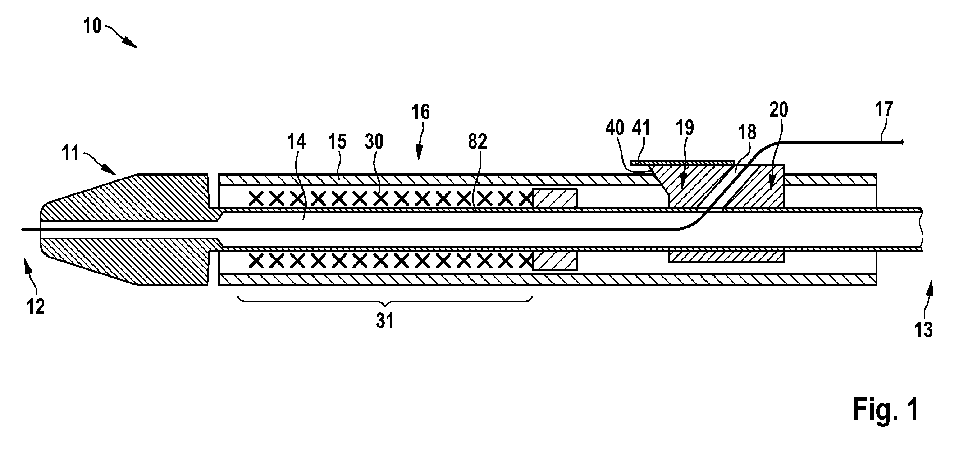

Fig. 1 zeigt eine bevorzugte Ausführungsform des Kathetersystems (10) zur Einführung eines selbstexpandierenden Stents (30). Das Kathetersystem (10) besteht aus einem Katheter (11) mit einem distalen (12) und einem proximalen (13) Ende. Der Katheter (11) hat einen Innenschaft (82) mit einem Führungsdrahtlumen (14) und einen Führungsdrahtausgang (18). Der Führungsdraht (17) verläuft am distalen Ende des Katheters (12) innerhalb des Führungsdrahtlumens (14). Durch den Führungsdrahtausgang (18) verlässt der Führungsdraht (17) das Führungsdrahtlumen (14) und läuft außerhalb des Katheters (11) weiter.

Ferner besteht das Kathetersystem (10) aus einem Funktionsabschnitt (31), der zur Aufnahme des selbstexpandierenden Stents (30) nahe dem distalen Ende des Katheters (12) dient. Über dem Funktionsabschnitt (31) wird zwecks Implantation ein gecrimpter, selbstexpandierenden Stent (30) angebracht. Durch die Crimpung wird er auf den kleinstmöglichen Durchmesser komprimiert und hat dadurch den für die Implantation nötigen geringen Querschnitt. Am Behandlungsort wird der selbstexpandierende Stent (30) dilatiert und löst sich vom Funktionsabschnitt (31) und damit vom gesamten Katheter.

Der selbstexpandierenden Stent (30) ist vollständig oder teilweise umgeben von einem Außenschlauch (15). Bei selbstexpandierenden Stents dient der Außenschlauch (15) der Beibehaltung des gecrimpten Zustands des Stents (30) während der Implantation. Am Behandlungsort wird der Außenschlauch (15) zurückgezogen und der Stent expandiert und nimmt seinen vorher eingeprägten dilatierten Zustand ein.

Der Führungsdrahtausgang (18) befindet sich nahe dem distalen Ende des Katheters (12) und proximal vom Funktionsabschnitt (31), hier vom Stentaufnahmeabschnitt. Der Führungsdrahtausgang (18) beschreibt einen Bereich am Katheter, der in seiner Mitte eine durchgehende Perforation von Innenschaft (82) und Außenschlauch (15) aufweist. Der Führungsdrahtausgang (18) hat ein distales (19) und ein proximales (20) Teilstück.

Am distalen Teilstück (19) des Führungsdrahtausgangs (18) befindet sich die erfindungsgemäße Schneidevorrichtung (40). Sie ist an dem Innenschaft (82) fixiert und ragt radial nach außen. Nach Erreichen des Implantationsortes wird der selbstexpandierende Stent (30) durch Zurückziehen des Außenschlauchs (15) freigesetzt. Gleichzeitig mit dem Zurückziehen trifft der Außenschlauch (15) auf die Schneidevorrichtung (40) und wird durchtrennt.

Da der Außenschlauch (15) aus einem relativ starren Material besteht, gibt er zwar genug Platz für den Führungsdraht (17) frei, ist jedoch so steif, dass er in einer rohrförmigen Gestalt verbleibt und mitsamt dem Katheter (11) schonend aus dem Körper gezogen werden kann.

Weiterhin ist in Fig. 1 schematisch die Schutzvorrichtung (41) gezeigt, die die Schneidevorrichtung (40) gegenüber der Umgebung, insbesondere der Gefäßwände, abschirmt.

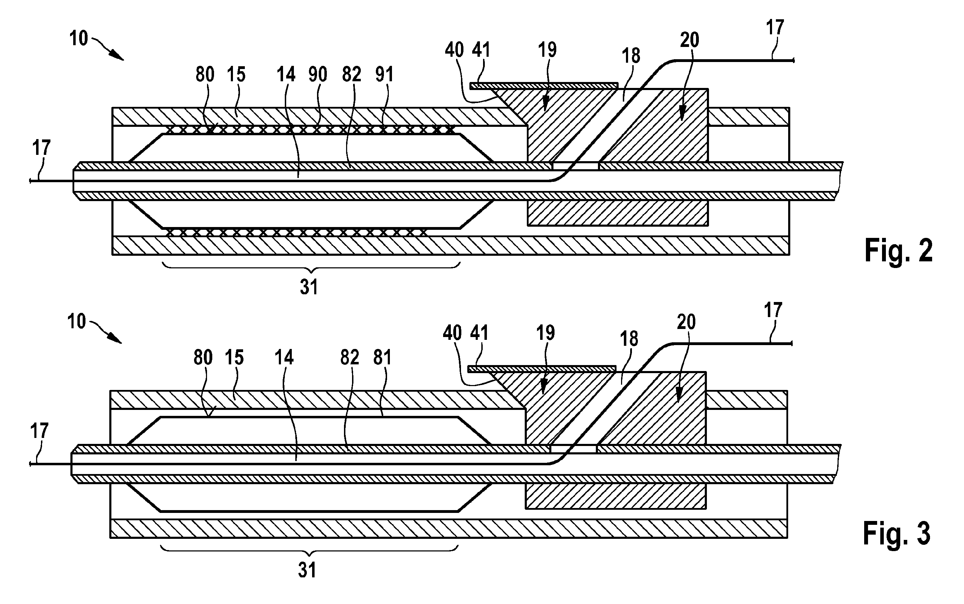

Fig. 2 zeigt eine weitere bevorzugte Ausführungsform des Kathetersystems (10). Der Katheter (11) dient zur Implantation eines ballondilatierbaren Stents (90). Der Aufbau ist analog zu dem in Fig. 1 beschriebenen Aufbau. Zusätzlich hat das in Fig. 2 gezeigte Kathetersystem einen Ballon (91), der zur Dilatation des Stents (90) am Behandlungsort dient. Er ist unterhalb des ballondilatierbaren Stents (90) und auf dem Außenschlauch (15) angebracht.

Analog zu Fig. 1 zeigt Fig. 2 diese weitere bevorzugte Ausführungsform des Kathetersystems (10) zur Einführung eines ballondilatierbaren Stents (90). Das Kathetersystem (10) besteht aus einem Katheter (11) mit einem distalen (12) und einem proximalen (13) Ende. Der Katheter (11) hat einen Innenschaft (82) mit einem Führungsdrahtlumen (14) und einen Führungsdrahtausgang (18). Der Führungsdraht (17) verläuft am distalen Ende des Katheters (12) innerhalb des Führungsdrahtlumens (14). Durch den Führungsdrahtausgang (18) verlässt der Führungsdraht (17) das Führungsdrahtlumen (14) und läuft außerhalb des Katheters (11) weiter.

Ferner besteht das Kathetersystem (10) aus einem Funktionsabschnitt (31), der zur Aufnahme des ballondilatierbaren Stents (90) nahe dem distalen Ende des Katheters (12) dient. Über dem Funktionsabschnitt (31) wird ein Ballon (91) angebracht. Auf diesem Ballon wird zwecks Implantation ein gecrimpter, ballondilatierbarer Stent (90) angebracht. Durch die Crimpung wird er auf den kleinstmöglichen Durchmesser komprimiert und hat dadurch den für die Implantation nötigen geringen Querschnitt. Am Behandlungsort wird der ballondilatierbare Stent (90) mit Hilfe des Ballons (91) dilatiert und löst sich vom Funktionsabschnitt (31) und damit vom gesamten Katheter.

Der ballondilatierbare Stent (90) ist vollständig oder teilweise umgeben von einem Außenschlauch (15). Der Außenschlauch (15) schützt ein auf der Stent-Oberfläche evtl. vorhandenes Medikament oder dient der Stent-Halterung auf dem Ballon (91) bzw. auf dem Katheter (10). Am Behandlungsort wird der Außenschlauch (15) zurückgezogen, der Ballon (91) dilatiert und damit der Stent expandiert.
Der Führungsdrahtausgang (18) befindet sich nahe dem distalen Ende des Katheters (12) und proximal vom Funktionsabschnitt (31), hier vom Stentaufnahmeabschnitt. Der Führungsdrahtausgang (18) beschreibt einen Bereich am Katheter, der in seiner Mitte eine durchgehende Perforation von Innenschaft (82) und Außenschlauch (15) aufweist. Der Führungsdrahtausgang (18) hat ein distales (19) und ein proximales (20) Teilstück.

Am distalen Teilstück (19) des Führungsdrahtausgangs (18) befindet sich die erfindungsgemäße Schneidevorrichtung (40). Sie ist an dem Innenschaft (82) fixiert und ragt radial nach außen. Nach Erreichen des Behandlungsortes wird der Außenschlauch (15) zurückgezogen und trifft dadurch auf die Schneidevorrichtung (40) und wird durchtrennt.

Da der Außenschlauch (15) aus einem relativ starren Material besteht, gibt er zwar genug Platz für den Führungsdraht (17) frei, ist jedoch so steif, dass er in einer rohrförmigen Gestalt verbleibt und mitsamt dem Katheter (11) schonend aus dem Körper gezogen werden kann.

Weiterhin ist in Fig. 2 schematisch die Schutzvorrichtung (41) gezeigt, die die Schneidevorrichtung (40) gegenüber der Umgebung, insbesondere der Gefäßwände, abschirmt.

Fig. 3 zeigt eine weitere bevorzugte Ausführungsform des Kathetersystems (10). In Fig. 3 ist der Funktionsabschnitt (31) als Ballonabschnitt ausgelegt. Auf dem Innenschaft (82) ist ein Ballon (81) angebracht, der eine Medikamenten-Beschichtung (80) aufweist. Der Ballon (81) ist vollständig oder teilweise umgeben von einem Außenschlauch (15). Der Außenschlauch (15) dient dem Schutz des Medikaments vor Ablösen von der Ballonoberfläche.

Am distalen Teilstück (19) des Führungsdrahtausgangs (18) befindet sich die erfindungsgemäße Schneidevorrichtung (40). Sie ist an dem Innenschaft (82) fixiert und ragt radial nach außen. Am Behandlungsort wird der Außenschlauch (15) zurückgezogen und durch die erfindungsgemäße Schneidevorrichtung (40) durchtrennt. Der Ballon (81) wird dilatiert. Solange er die Wände des Gefäßes berührt, gibt er das Medikament an die Gefäßwand ab.

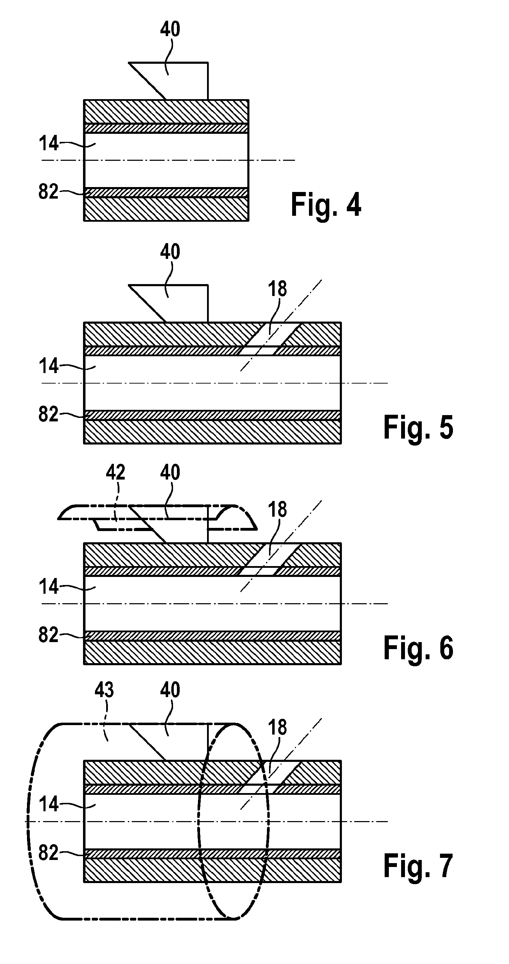

In Fig. 4 ist die Schneidevorrichtung (40) in einer Detailvergrößerung dargestellt. Die Schneidevorrichtung (40) kann unabhängig vom Führungsdrahtausgang (18) am Katheter angeordnet sein. Einzige Voraussetzung ist, dass die Schneidevorrichtung (40) distal vom Führungsdrahtausgang (18) angeordnet ist.

Fig. 5 zeigt die Kombination von Schneidevorrichtung (40) und Führungsdrahtausgang (18) in einem Bauteil. Hier liegen Schneidevorrichtung (40) und Führungsdrahtausgang (18) relativ nah beieinander und sind durch ein Bauteil fixiert.

In den Figuren 6 und 7 sind in einer Detailvergrößerung verschiedene Ausgestaltungen der Schutzvorrichtung (41) dargestellt. Fig. 6 zeigt eine Schutzvorrichtung in Form einer Halbschale (42), die direkt auf der Schneidevorrichtung (40) angebracht ist und diese umschließt. Fig. 7 zeigt schematisch eine Schutzvorrichtung in Form eines Rohrs (43). Das Rohr (43) ist derart angebracht, dass es den kompletten Katheter (11) im Bereich der Schneidevorrichtung (40) umschließt. Der Katheter (11) verläuft axial durch das Rohr (43); und das Rohr (43) hat einen Durchmesser, der exakt dem Durchmesser des Katheters (11) mit der Schneidevorrichtung (40) entspricht.

### Bezugszeichenliste:

- 10: Kathetersystem
- 11: Katheter
- 12: distales Ende des Katheters
- 13: proximales Ende des Katheters
- 14: Führungsdrahtlumen
- 82: Innenschaft
- 15: Außenschlauch
- 16: Außenbereich des Katheters
- 17: Führungsdraht
- 18: Führungsdrahtausgang
- 19: distales Teilstück des Führungsdrahtausgangs
- 20: proximales Teilstück des Führungsdrahtausgangs
- 30: selbstexpandierender Stent
- 31: Funktionsabschnitt
- 40: Schneidevorrichtung
- 41: Schutzvorrichtung
- 42: Schutzvorrichtung als Halbschale
- 43: Schutzvorrichtung als Rohr
- 80: medikamentenbeschichteter Ballon
- 81: Medikamenten-Beschichtung
- 90: ballondilatierbarer Stent
- 91: Ballon

## Patentansprüche

1. Kathetersystem (10) bestehend aus
- einem Katheter (11) mit einem distalen (12) und einem proximalen (13) Ende,
- einem Funktionsabschnitt (31), der nahe dem distalen Ende des Katheters (12) liegt,
- einem Innenschaft (82) mit Führungsdrahtlumen (14),
- einem Außenschlauch (15), wobei der Außenschlauch (15) den Funktionsabschnitt (31) vollständig oder teilweise umschließt,
- einem Führungsdrahtausgang (18) mit distalem (19) und proximalem (20) Teilstück, wobei der Führungsdrahtausgang (18) durch den Innenschaft (82) und den Außenschlauch (15) führt,
- einem Führungsdraht (17), wobei sich der Führungsdraht (17) am distalen Ende des Katheters (12) innerhalb des Führungsdrahtlumens (14) befindet, und durch den Führungsdrahtausgang (18) aus dem Katheter (11) herausgeführt wird, wobei der Führungsdrahtausgang (18) nahe dem distalen Ende des Katheters (12) und proximal vom Funktionsabschnitt (31) angebracht ist,
**dadurch gekennzeichnet, dass**
an dem distalen Teilstück (19) des Führungsdrahtausgangs (18) eine Schneidevorrichtung (40) zum Durchtrennen des Außenschlauchs (15) beim Zurückziehen desselben angebracht ist.

2. Kathetersystem (10) nach Anspruch 1, **dadurch gekennzeichnet, dass** die Schneidevorrichtung (40) an dem Innenschaft (82) fixiert ist.

3. Kathetersystem (10) nach Anspruch 1 oder 2, **dadurch gekennzeichnet, dass** die Schneidevorrichtung (40) in Form einer Klinge ausgestaltet ist.

4. Kathetersystem (10) nach einem der Ansprüche 1 bis 3, **dadurch gekennzeichnet, dass** über der Schneidevorrichtung (40) eine Schutzvorrichtung (41) angebracht ist.

5. Kathetersystem (10) nach Anspruch 4, **dadurch gekennzeichnet, dass** die Schutzvorrichtung (41) die Form einer Halbschale (42) aufweist.

6. Kathetersystem (10) nach Anspruch 4, **dadurch gekennzeichnet, dass** die Schutzvorrichtung (41) die Form eines Rohrs (43) aufweist.

7. Kathetersystem (10) nach einem der Ansprüche 1 bis 6, **dadurch gekennzeichnet, dass** der Funktionsabschnitt (31) ausgelegt ist als Stent-Aufnahmeabschnitt.

8. Kathetersystem (10) nach einem der Ansprüche 1 bis 6, **dadurch gekennzeichnet, dass** der Funktionsabschnitt (31) ausgelegt ist als Ballonabschnitt.

9. Kathetersystem (10) nach Anspruch 7, **dadurch gekennzeichnet, dass** der Stent selbst expandierbar oder ballonexpandierbar ist.
